# EUROPEAN PATENT SPECIFICATION

(11) **EP 2 934 453 B1**
(45) Date of publication and mention of the grant of the patent: **23.08.2017**
(21) Application number: 12824835.8
(22) Date of filing: 21.12.2012
(51) Int. Cl.: A61K 8/29, A61Q 11/00, A61K 8/49, A61K 8/19, A61K 8/81, A61K 8/46

(54) **NON-STAINING TOOTHPASTE**
NICHTFÄRBENDE ZAHNPASTA
DENTIFRICE NON-COLORANT

(43) Date of publication of application: 28.10.2015
(73) Proprietor: Colgate-Palmolive Company, New York, NY 10022 (US)
(72) Inventor: SZEWCZYK, Gregory, Flemington, New Jersey 08822 (US); PATEL, Neeta Atul, Monmouth Junction, New Jersey 08852 (US); JOGUN, Suzanne, Wayne, New Jersey 07470 (US)
(74) Representative: Wibbelmann, Jobst
(86) International application number: PCT/US2012/071137
(87) International publication number: WO 2014/098881

(56) References cited:
- EP-A1- 1 935 395
- EP-A1- 2 277 982
- US-A- 5 032 386
- US-A1- 2012 070 478

## Description

### BACKGROUND OF THE INVENTION

It is recommended that children should brush their teeth for at least 45-60 seconds, and adults for at least 90 to 120 seconds. Most people, especially children, do not brush their teeth for a sufficient period of time to obtain maximum benefit, and moreover have difficulty accurately estimating the time necessary to brush the teeth. Toothpaste formulations have been developed which comprise dissolvable films or microcapsules comprising pigment, which are designed to release the pigment after 30 to 120 seconds of brushing, providing a color change signal of adequate brushing. One problem with these formulations, however, is the tendency of the pigment to stain the bristles of the toothbrush. There is a need for improved formulations with reduced potential to stain the bristles, but still providing a clear signal upon adequate brushing.

US-A-2012/0070478 discloses colour changing consumer products. EP-A-2277982 discloses a colour changing composition. EP-A-1935395 discloses an oral composition. US-A-5,032,386 discloses an antiplaque antibacterial composition.

### BRIEF SUMMARY OF THE INVENTION

Following systematic testing, varying different formulation components, we surprisingly found that an interaction exists between the polymer thickener (in this case Gantrez) and titanium dioxide (a common opaque white pigment) that drives the deposit of the colored (blue) pigment on the bristles. Unexpectedly, the lower the TiO₂ level the less the blue pigment appears to deposit on the bristles. Without being bound by theory, we hypothesize that this is driven by a charge (polarization) phenomena.

The invention thus provides a dentifrice according to claim 1, and a dentifrice for use according to claim 8. Preferred features are defined in the dependent claims.

Further areas of applicability of the present invention will become apparent from the detailed description provided hereinafter. It should be understood that the detailed description and specific examples, while indicating the preferred embodiment of the invention, are intended for purposes of illustration only and are not intended to limit the scope of the invention.

### DETAILED DESCRIPTION OF THE INVENTION

The following description of the preferred embodiment(s) is merely exemplary in nature and is in no way intended to limit the invention, its application, or uses.

The invention thus provides, in a first embodiment, a dentifrice (Dentifrice 1) having a pigment system which comprises one or more pigments and titanium dioxide, wherein the level of titanium dioxide is 0.05-0.2% by weight of the dentifrice, wherein the one or more pigments is Pigment Blue 15: 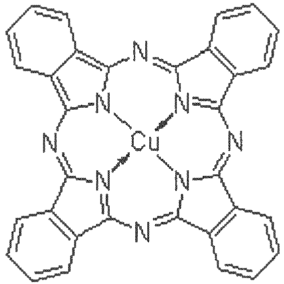 and wherein the dentifrice comprises 1.5 - 2.0% of sodium lauryl sulfate and 0.5 - 2.0% of a copolymer of methyl vinyl ether and maleic anhydride. For example:
1.1. Any of the foregoing dentifrice wherein the one or more pigments are present in an amount from about 0.02 to about 0.5% of the total weight of the composition.
1.2. The immediately preceding dentifrice wherein the one or more pigments are present in an amount from about 0.05 to about 0.3% of the total weight of the composition.
1.3. Any of the foregoing dentifrices wherein the one or more pigments are releasable during brushing with an oral care implement.
1.4. The immediately preceding dentifrice wherein a dissolvable or disintegratable film comprises the one or more releasable pigments which are released during brushing.
1.5. Any of the foregoing dentifrices wherein the releasable pigment provides a color signal to the user of adequate brushing and the titanium dioxide reduces or inhibits the staining of the oral care implement by the releasable pigment
1.6. Any of the foregoing dentifrices wherein the oral care implement is a toothbrush.
1.7. Any of the foregoing dentifrices wherein the amount of titanium dioxide is about 0.1%.
1.8. Any of the foregoing dentifrices comprising a copolymer of methyl vinyl ether and maleic anhydride having a molecular weight (M.W.) of about 30,000 to about 1,000,000, most preferably about 30,000 to about 800,000 (for example as sold under the tradename Gantrez® e.g., AN 139 (M.W. 500,000), AN 119 (M.W. 250,000) and preferably S-97 Pharmaceutical Grade (M.W. 700,000) available from ISP Technologies, Inc., Bound Brook, N.J. 08805), e.g., in an amount of 1 - 2%.
1.9. Any of the foregoing dentifrices wherein the dentifrice further comprises an effective amount of an antibacterial agent, e.g., triclosan, e.g., about 0.3%; an anionic surfactant, e.g., sodium lauryl sulfate (SLS), e.g. 1.5-2%, an effective amount of a fluoride source, e.g., a soluble fluoride salt, e.g. sodium fluoride, about 0.24%; humectants, e.g. comprising one or more of glycerin, sorbitol, and propylene glycol, e.g., 45-75%; thickeners, e.g., selected from xanthan gum, carrageenan, and colloidal silica, e.g., 1-10 %; an anionic polymer, e.g., a copolymer of methyl vinyl ether and maleic anhydride (for example, Gantrez®), e.g., 0.5-3%, e.g. about 2%, and water, e.g., 15-25%, e.g., about 20%.
1.10. Any of the foregoing dentifrices wherein the releasable pigment is substantially released after a period of greater than 30 seconds and less than 180 seconds of brushing in the presence of water, e.g., about 45-60 seconds in a toothpaste for use by a child and about 90-120 seconds in a toothpaste for use by an adult; wherein the release of the releasable pigment provides a color signal to the user of adequate brushing.
1.11. Any of the foregoing dentifrices wherein the releasable pigment does not significantly stain the bristles of the toothbrush during use.

Also described herein is a method of cleaning the teeth comprising brushing with a dentifrice having a pigment system which comprises (i) a dissolvable or disintegratable film comprising one or more releasable pigments which are released during brushing, and (ii) titanium dioxide, e.g., any of Dentifrice 1, *et seq.,* for example
a. the method wherein the brushing is continued until the film disintegrates and the pigment provides a color signal to the user of adequate brushing, for example,
b. the foregoing method when the brushing time before the film disinegrates is between 30 and 180 seconds, e.g., about 45-60 seconds for a toothpaste for use by a child and about 90-120 seconds for a toothpaste for use by an adult.

*Orally acceptable:* The compositions of the invention are intended for topical use in the mouth, thus components for use in the present invention should be orally acceptable, that is, safe for topical use in the mouth, in the amounts and concentrations provided.

As used throughout, ranges are used as shorthand for describing each and every value that is within the range. Any value within the range can be selected as the terminus of the range. In the event of a conflict in a definition in the present disclosure and that of a cited reference, the present disclosure controls.

Unless otherwise specified, all percentages and amounts expressed herein and elsewhere in the specification should be understood to refer to percentages by weight. The amounts given are based on the active weight of the material.

Embodiments of the present invention are further described in the following examples. The examples are merely illustrative and do not in any way limit the scope of the invention as described and claimed.

### EXAMPLES

### Example 1 - Formulation optimization

In initial experiments wherein toothpaste was prepared by admixing dissolvable film flakes comprising Pigment Blue 15 in a modified commercial dentifrice base, the toothbrush bristles are stained blue following repeated use and release of the pigment. The amount of blue on the bristles is measured using Lab color space (a color-opponent space with dimension L for lightness and a and b for the color-opponent dimensions, based on nonlinearly compressed CIE XYZ color space coordinates).

Initial experiments are performed with a variety of formulations to determine the key formulation drivers of bristle staining. A Design of Experiments approach is utilized to refine and systematically optimize the formulation, focusing on three ingredients.

Twenty-five formulations were prepared, with variables within this framework, and are systematically assessed for performance and bristle staining. The formulations containing Gantrez (a copolymer of methyl vinyl ether and maleic anhydride), sodium lauryl sulfate (SLS) and titanium dioxide within the above ranges follow below in Table 1:

**Table 1**

| *Formulations* | %w/w |
|---|---|
| GANTREZ® S-97 (a copolymer of methyl vinyl ether and maleic anhydride) | 0.50 - 2.00 |
| TITANIUM DIOXIDE | 0.01 - 0.375 |
| SODIUM LAURYL SULFATE (SLS) | 1.50 - 2.00 |
| WATER | 18.10 |
| SODIUM FLUORIDE | 0.24 |
| SODIUM SACCHARIN | 0.30 |
| GLYCERIN | 29.73-32.11 |
| XANTHAN GUM | 0.50 |
| IOTA CARRAGEENAN | 0.50 |
| SORBITOL | 20.85 |
| PROPYLENE GLYCOL | 0.50 |
| NaOH, 50% SOLUTION | 1.20 |
| DENTAL TYPE SILICA-ZEODENT 105-HIGH CLEANING SILICA | 10.00 |
| DENTAL TYPE SILICA (ZEODENT 115) ABRASIVE | 8.50 |
| DENTAL TYPE SILICA-ZEODENT 165-SYNTH. AMORPHOUS | 3.00 |
| MICA | 0.70 |
| FILM COMPRISING PIGMENT BLUE 15 | 0.20 |
| TRICLOSAN | 0.30 |
| FLAVOR | 1.00 |
| Total | 100.00 |

It is surprisingly found that an interaction exists between Gantrez (a copolymer of methyl vinyl ether and maleic anhydride) and titanium dioxide that can cause deposit of the blue pigment on the bristles. Unexpectedly, the lower the TiO₂ level, the less the blue pigment appears to deposit on the bristles. It is hypothesized that deposition may be driven by a charge (polarization) phenomena.

An optimized formulation is prepared, based on this data in Table 2 below:

**Table 2**

| | %w/w |
|---|---|
| GANTREZ S-97 (a copolymer of methyl vinyl ether and maleic anhydride) | 2.0 |
| TITANIUM DIOXIDE | 0.1 |
| SODIUM LAURYL SULFATE (SLS) | 1.75 |
| WATER | 18.10 |
| SODIUM FLUORIDE | 0.24 |
| SODIUM SACCHARIN | 0.30 |
| GLYCERIN | 32.11 |
| XANTHAN GUM | 0.50 |
| IOTA CARRAGEENAN | 0.50 |
| SORBITOL | 20.85 |
| PROPYLENE GLYCOL | 0.50 |
| NaOH, 50% SOLUTION | 1.20 |
| DENTAL TYPE SILICA (ZEODENT 105) HIGH CLEANING SILICA | 10.00 |
| DENTAL TYPE SILICA (ZEODENT 115) ABRASIVE | 8.50 |
| DENTAL TYPE SILICA (ZEODENT 165) SYNTH. AMORPHOUS | 3.00 |
| MICA | 0.70 |
| FILM COMPRISING PIGMENT BLUE 15 | 0.20 |
| TRICLOSAN | 0.30 |
| FLAVOR | 1.00 |
| Total | 100.00 |

This formulation is tested for whether the toothbrush bristles are stained following use. Whereas the original formulation showed substantial bristle staining (ΔE > 20), the optimized formulation, while providing a good color change upon brushing, showed very little bristle staining (ΔE < 5).

As those skilled in the art will appreciate, numerous changes and modifications may be made to the embodiments described herein without departing from the invention. It is intended that all such variations fall within the scope of the appended claims.

## Claims

1. A dentifrice having a pigment system which comprises one or more pigments, and titanium dioxide, wherein the level of titanium dioxide is 0.05 - 0.2% by weight of the dentifrice, wherein the one or more pigment is Pigment Blue 15: 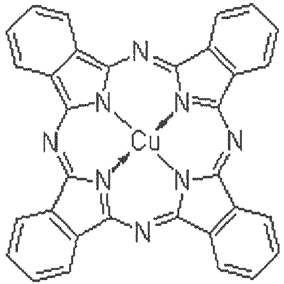 and wherein the dentifrice comprises 1.5 - 2.0% of sodium lauryl sulfate and 0.5 - 2.0% of a copolymer of methyl vinyl ether and maleic anhydride.

2. The dentifrice of claim 1, wherein the one or more pigments are releasable during brushing with an oral care implement.

3. The dentifrice of claim 1 or 2, wherein the releasable pigment provides a color signal of adequate brushing to the user and the titanium dioxide reduces or inhibits the staining of the oral care implement by the releasable pigment.

4. The dentifrice of any of the foregoing claims, wherein a dissolvable or disintegratable film comprises the one or more releasable pigments which are released during brushing.

5. The dentifrice of any of the foregoing claims wherein the one or more releasable pigments comprises an insoluble pigment.

6. The dentifrice of any of the foregoing claims comprising
a) about 0.1 % titanium dioxide
b) about 0.3% triclosan,
c) 1.5-2% sodium lauryl sulfate (SLS),
d) about 0.24% sodium fluoride,
e) 45-75% humectants comprising one or more of glycerin, sorbitol, and propylene glycol,
f) 1-10 % thickeners comprising one or more of xanthan gum, carrageenan, and colloidal silica,
g) 0.5-3% of a copolymer of methyl vinyl ether and maleic anhydride, and
h) 15-25% water.

7. The dentifrice of any of the foregoing claims, wherein the releasable pigment is substantially released after a period of greater than 30 seconds and less than 180 seconds of brushing in the presence of water, wherein the release of the releasable pigment provides a color signal to the user of adequate brushing.

8. The dentifrice of any of claims 1-7, for use in a method of cleaning teeth.

9. The dentifrice for use according to claim 8, wherein the pigment provides a color signal of adequate brushing with an oral care implement to the user and the titanium dioxide reduces or inhibits the staining of the oral care implement by the releasable pigment.

10. The dentifrice for use according to claim 9, wherein the brushing time before the film dissolves or disintegrates is between 30 and 180 seconds.

11. The dentifrice for use according to claim 9 or claim 10, wherein the oral care implement is a toothbrush and the titanium dioxide reduces or inhibits staining bristles of the toothbrush during brushing.

## Patentansprüche

1. Zahnreinigungsmittel mit einem Pigmentsystem, umfassend ein oder mehrere Pigmente und Titandioxid, worin die Konzentration des Titandioxids 0,05 - 0,2 Gew.-% des Zahnreinigungsmittels ist, worin das eine oder mehrere Pigmente Pigment Blau 15 ist: 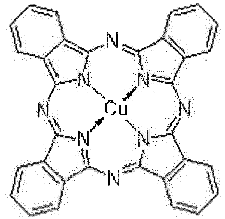 und worin das Zahnreinigungsmittel 1,5 - 2,0 % von Natriumlaurylsulfat und 0,5 - 2,0 % eines Copolymers aus Methylvinylether und Maleinsäure Anhydrid umfasst.

2. Zahnreinigungsmittel nach Anspruch 1, worin das eine oder mehrere Pigmente während des Bürstens mit einem Mundpflegewerkzeug freisetzbar sind.

3. Zahnreinigungsmittel nach Anspruch 1 oder 2, worin das freisetzbare Pigment ein Farbsignal bei ausreichendem Bürsten an den Verwender bereitstellt und das Titandioxid die Einfärbung des Mundpflegewerkzeugs durch das freisetzbare Pigment vermindert oder inhibiert.

4. Zahnreinigungsmittel nach irgendeinem der vorhergehenden Ansprüche, worin der auflösbare oder zersetzbare Film ein oder mehrere freisetzbare Pigmente umfasst, die während des Bürstens freigesetzt werden.

5. Zahnreinigungsmittel nach irgendeinem der vorhergehenden Ansprüche, worin das eine oder mehrere freisetzbare Pigmente ein unlösliches Pigment umfasst.

6. Zahnreinigungsmittel nach irgendeinem der vorhergehenden Ansprüche umfassend
a) etwa 0,1 % Titandioxid,
b) etwa 0,3 % Triclosan,
c) 1,5 - 2 %Natriumlaurylsulfat (SLS),
d) etwa 0,24 % Natriumfluorid,
e) 45 - 75 % Befeuchtungsmittel umfassend ein oder mehrere aus Glycerin, Sorbitol und Propylenglycol,
f) 1 - 10 % Verdickungsmittel umfassend ein oder mehrere aus Xanthangummi, Carrageenan und kolloidalem Silica,
g) 0,5 - 3 % eines Copolymers aus Methylvinylether und Maleinsäureanhydrid, und
h) 15 - 25 % Wasser.

7. Zahnreinigungsmittel nach irgendeinem der vorhergehenden Ansprüche, worin das freisetzbare Pigment im Wesentlichen nach einer Periode von größer als 30 Sekunden und weniger als 180 Sekunden Bürsten in Gegenwart von Wasser abgegeben wird, worin die Abgabe des abgebbaren Pigments ein Farbsignal an Verwender bei ausreichenden Bürsten bereitstellt.

8. Zahnreinigungsmittel nach irgendeinem der Ansprüche 1 bis 7 zur Verwendung in einem Verfahren zur Reinigung von Zahn.

9. Zahnreinigungsmittel zur Verwendung gemäß Anspruch 8, worin das Pigment ein Farbsignal für ausreichendes Bürsten mit einem Mundpflegewerkzeug für den Verwender bereitstellt und das Titandioxid die Einfärbung des Mundpflegewerkzeugs durch das abgebbare Pigment reduziert oder inhibiert.

10. Zahnreinigungsmittel zur Verwendung gemäß Anspruch 9, worin die Bürstenzeit bevor der Film sich auflöst oder zersetzt zwischen 30 und 180 Sekunden ist.

11. Zahnreinigungsmittel zur Verwendung nach Anspruch 9 oder Anspruch 10, worin das Mundpflegewerkzeug eine Zahnbürste ist und das Titandioxid die Einfärbung der Borsten der Zahnbürste während des Bürstens reduziert oder inhibiert.

## Revendications

1. Dentifrice ayant un système de pigments qui comprend un ou plusieurs pigments, et du dioxyde de titane, dans lequel le taux de dioxyde de titane est de 0,05 à 0,2 % en poids du dentifrice, dans lequel le ou les pigments sont le Pigment Blue 15 : 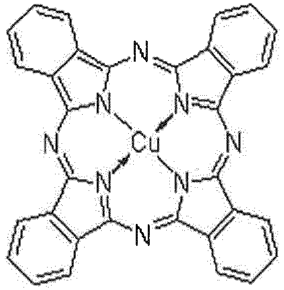 et dans lequel le dentifrice comprend 1,5 à 2,0 % de laurylsulfate de sodium et 0,5 à 2,0 % d'un copolymère d'éther de méthyle et de vinyle et d'anhydride maléique.

2. Dentifrice selon la revendication 1, dans lequel le ou les pigments peuvent être libérés pendant le brossage avec un instrument de soin buccal.

3. Dentifrice selon la revendication 1 ou 2, dans lequel le pigment pouvant être libéré fournit un signal de couleur de brossage adéquat à l'utilisateur, et le dioxyde de titane réduit ou inhibe la coloration de l'instrument de soin buccal par le pigment pouvant être libéré.

4. Dentifrice selon l'une quelconque des revendications précédentes, dans lequel un film dissoluble ou désintégrable comprend le ou les pigments pouvant être libérés qui sont libérés pendant le brossage.

5. Dentifrice selon l'une quelconque des revendications précédentes, dans lequel le ou les pigments pouvant être libérés comprennent un pigment insoluble.

6. Dentifrice selon l'une quelconque des revendications précédentes, comprenant
a) environ 0,1 % de dioxyde de titane
b) environ 0,3 % de triclosan,
c) 1,5 à 2 % de laurylsulfate de sodium (SLS),
d) environ 0,24 % de fluorure de sodium,
e) 45 à 75 % d'humectants comprenant un ou plusieurs humectants parmi la glycérine, le sorbitol et le propylène glycol,
f) 1 à 10 % d'épaississants comprenant un ou plusieurs épaississants parmi la gomme de xanthane, le carraghénane et la silice colloïdale,
g) 0,5 à 3 % d'un copolymère d'éther de méthyle et de vinyle et d'anhydride maléique, et
h) 15 à 25 % d'eau.

7. Dentifrice selon l'une quelconque des revendications précédentes, dans lequel le pigment pouvant être libéré est libéré sensiblement après une période supérieure à 30 secondes et inférieure à 180 secondes de brossage en présence d'eau, dans lequel la libération du pigment pouvant être libéré fournit un signal de couleur à l'utilisateur d'un brossage adéquat.

8. Dentifrice selon l'une quelconque des revendications 1 à 7, pour une utilisation dans un procédé de nettoyage des dents.

9. Dentifrice pour une utilisation selon la revendication 8, dans lequel le pigment fournit un signal de couleur d'un brossage adéquat avec un instrument de soin buccal à l'utilisateur, et le dioxyde de titane réduit ou inhibe la coloration de l'instrument de soin buccal par le pigment pouvant être libéré.

10. Dentifrice pour une utilisation selon la revendication 9, dans lequel le temps de brossage avant que le film ne se dissolve ou se désagrège est compris entre 30 et 180 secondes.

11. Dentifrice pour une utilisation selon la revendication 9 ou la revendication 10, dans lequel l'instrument de soin buccal est une brosse à dents, et le dioxyde de titane réduit ou inhibe la coloration des poils de la brosse à dents pendant le brossage.
